# EUROPEAN PATENT APPLICATION

(11) **EP 4 339 297 A1**
(43) Date of publication of application: **20.03.2024**
(21) Application number: 22196263.2
(22) Date of filing: 19.09.2022
(51) Int. Cl.: C12Q 1/6841, C12Q 1/6827, C12Q 1/6886

(54) **TARGETED DETECTION OF SINGLE NUCLEOTIDE POLYMORPHISMS (SNP) DIRECTLY ON TISSUE**

(71) Applicant: Miltenyi Biotec B.V. & Co. KG, 51429 Bergisch Gladbach (DE)
(72) Inventor: NEHME, Chris, 51429 Bergisch Gladbach (DE); PINARD, Robert, 51429 Bergisch Gladbach (DE); MEYER, Hansueli, 51429 Bergisch Gladbach (DE)
(74) Representative: Kisters, Michael Marcus

(57) **Abstract**

The invention is directed to a method for determining the sequence of single nucleotide polymorphisms (SNP) of at least one RNA strand on tissue samples **characterized by** the steps
a. providing an oligonucleotide comprising 50 - 1000 nucleotides, having a 3' end and a 5' end each with 10 to 30 nucleic acids complementary to the nucleic acids of the RNA from the SNP towards the 3' end and the 5' end of the RNA respectively, a primer region and at least one detection region, each having 2 to 20 nucleotides
b. hybridizing the oligonucleotide with its 5' and 3' ends to complementary parts of the at least one RNA strand to create a padlock with a gap of one nucleotide between the 5' and the 3' end of the oligonucleotide thereby bracketing the SNP
c. performing for at least one of the nucleotides Adenine (A), Thymine (T), Cytosine (C), Guanine (G) and Uracil (U) a sequence comprising (i) filling the gap of the padlock with the at least one nucleotide, thereby generating a single strand circular template; (ii) multiplying the single strand circular template by a polymerase capable of rolling circle amplification using the primer region into a plurality of concatemers forming a rolony; (iii) binding to the detection regions at least one detection probe comprising a dye and a sequence of nucleotides complementary to the detection regions
d. detecting the dye of the detection probes, thereby determining the sequence of the single strand circular template

Use of the method for determination if one or more known single nucleotide polymorphisms (SNP) of RNA strands on a tissue sample are present.

## Description

The present invention is directed to a method for detecting the sequence and/or presence of single nucleotide polymorphisms (SNP) directly on tissue samples, optional including the spatial location of the SNP on the sample.

### BACKGROUND

SNPs are mutations in RNA of a single nucleotide, which might or might not affect the function of the RNA. In general, the sequence of the RNA is known and the position ond optionally the nature of the of the mutation are also known.

In case the SNP is known, they might be used as indicator of certain diseases like cancer. In such cases, the sequence is needs the be detected, but it is of greater interest to detect the presence or absence of the SNP, i.e. at best to detect the presence or absence of the disease.

Other SNPs are not known and it is of interest to sequence them besides detecting the presence or absence of the SNP.

In order to detect SNP's in a tissue sample, while maintaining spatial context, one must execute complicated, multi-step workflows that involve spatial molecular tagging, extraction, amplification and ex-situ sequencing of mRNA. In addition to being tedious, such workflows have the potential to introduce errors and lower confidence in results at multiple stage.

In general, detection of the location of mRNA in situ is known for example by US8551710B2. This method cannot detect and identify SNP variants.

Further, in situ nucleic acid sequencing of expanded biological samples is known for for Spatial Variant Detection from US10563257B2 or US2019/0241950A1. Drawback of these methods is that full sequencing on expanded tissue needs to be performed, which involves tedious and non-targeted workflow.

So far, no dedicated workflows exist for the targeted detection of single nucleotide polymorphisms (SNP) directly on tissue samples. Further it would be of interest to find out where SNP mutations are located spatially on a sample.

### SUMMARY OF THE INVENTION

Object of the invention is a method of directly detecting SNPs on a tissue sample, identifying the exact variants/substitutions present, without the need for mRNA extraction nor ex-situ sequencing, optionally including the spatial location of SNP's.

The method of the invention is directed to determining the sequence of single nucleotide polymorphisms (SNP) of at least one RNA strand on tissue samples characterized by the steps
a. providing an oligonucleotide comprising 50 - 1000 nucleotides, having a 3' end and a 5' end each with 10 to 30 nucleic acids complementary to the nucleic acids of the RNA from the SNP towards the 3' end and the 5' end of the RNA respectively, a primer region and at least one detection region, each having 2 to 20 nucleotides
b. hybridizing the oligonucleotide with its 5' and 3' ends to complementary parts of the at least one RNA strand to create a padlock with a gap of one nucleotide between the 5' and the 3' end of the oligonucleotide thereby bracketing the SNP
c. performing for at least one of the nucleotides Adenine (A), Thymine (T), Cytosine (C), Guanine (G) and Uracil (U) a sequence comprising (i) filling the gap of the padlock with the at least one nucleotide, thereby generating a single strand circular template; (ii) multiplying the single strand circular template by a polymerase capable of rolling circle amplification using the primer region into a plurality of concatemers forming a rolony; (iii) binding to the detection regions at least one detection probe comprising a dye and a sequence of nucleotides complementary to the detection regions
d. detecting the dye of the detection probes, thereby determining the sequence of the single strand circular template

In a first embodiment, by detecting the detection probes, the spatial location of the rolonies is determined.

It is further an object of the invention to use the method for determination if one or more either known or suspected or unknown single nucleotide polymorphisms (SNP) of RNA strands on a tissue sample are present

### LEGENDS TO THE DRAWINGS

The drawings shall explain the method of the invention and its embodiments without limiting the scope of the claims.

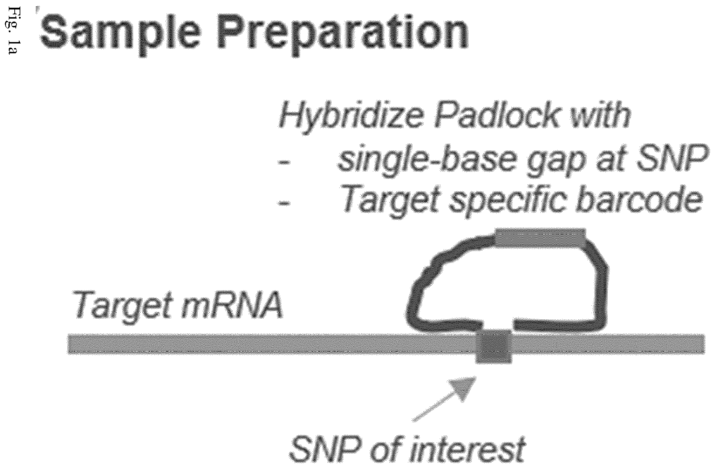

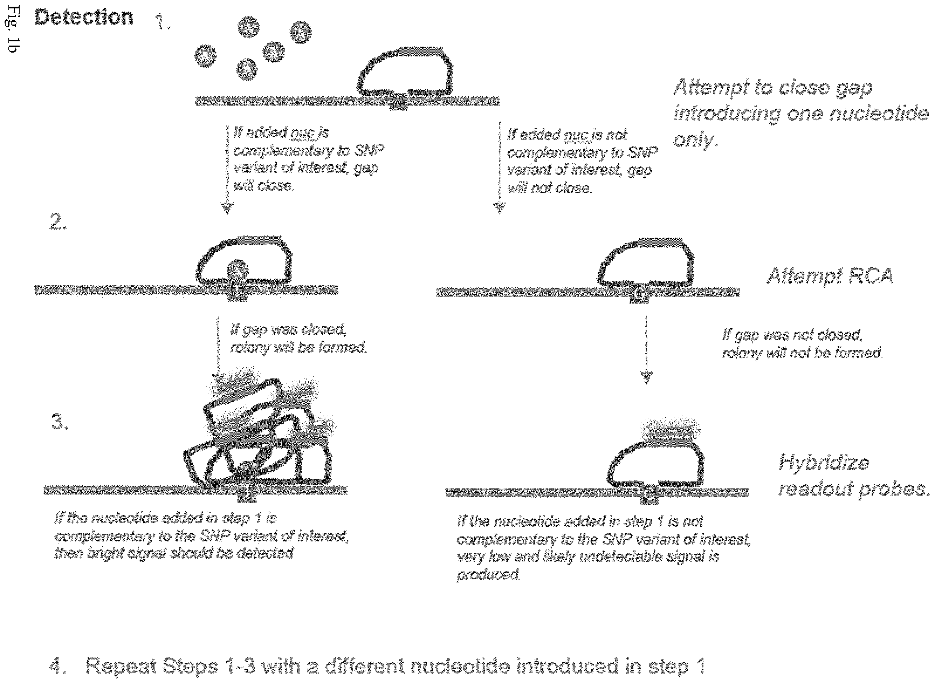

Figure 1 a/b and 9 show sketches of the proposed method.

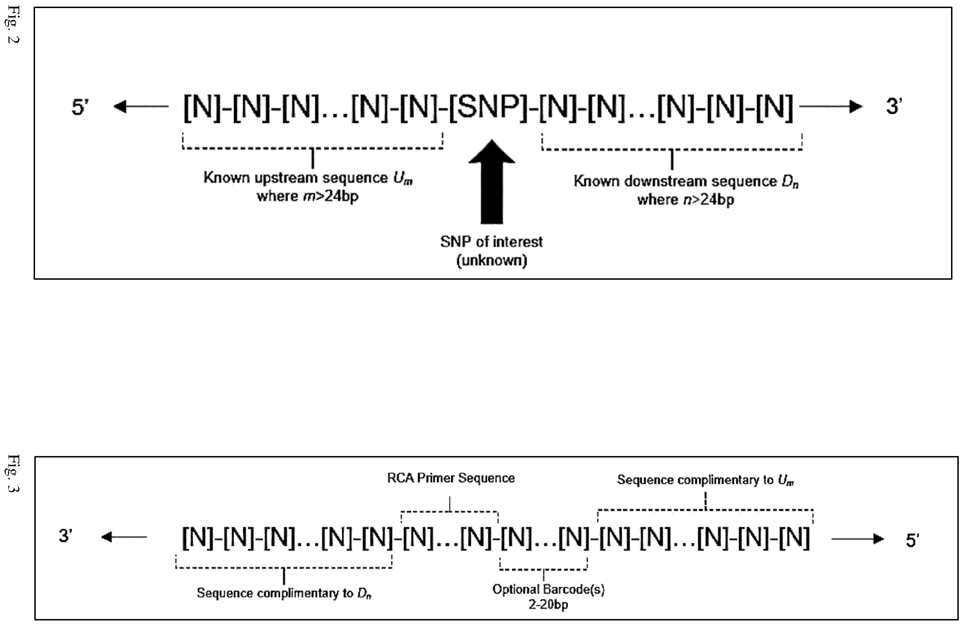

Figure 2 shows the prerequisite knowledge of target SNP of interest:
Figure 3 shows the structure of the provided oligonucleotide

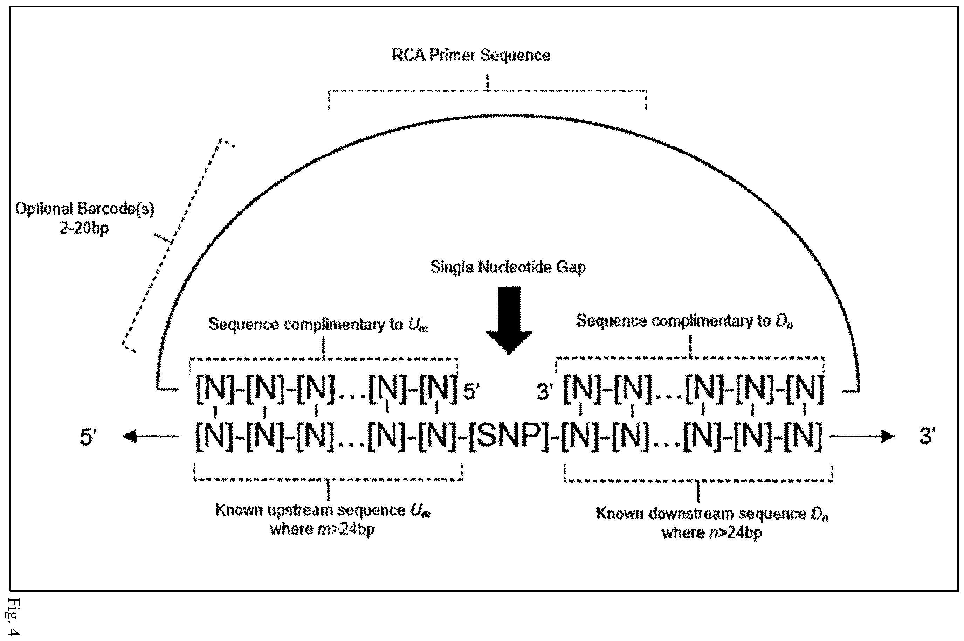

Figure 4 shows the resulting padlock product of the hybridization

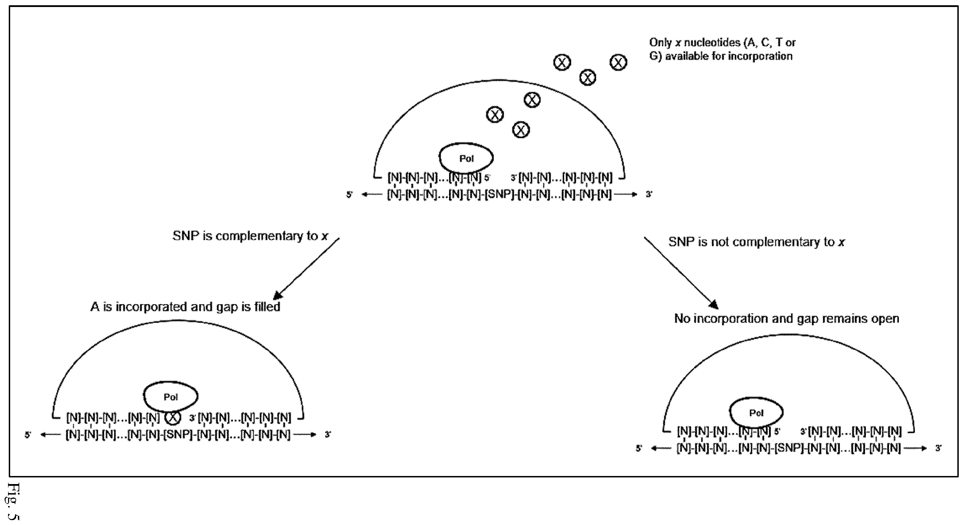

Figure 5 shows an example of a gap filling reaction by single base extension with a DNA Polymerase

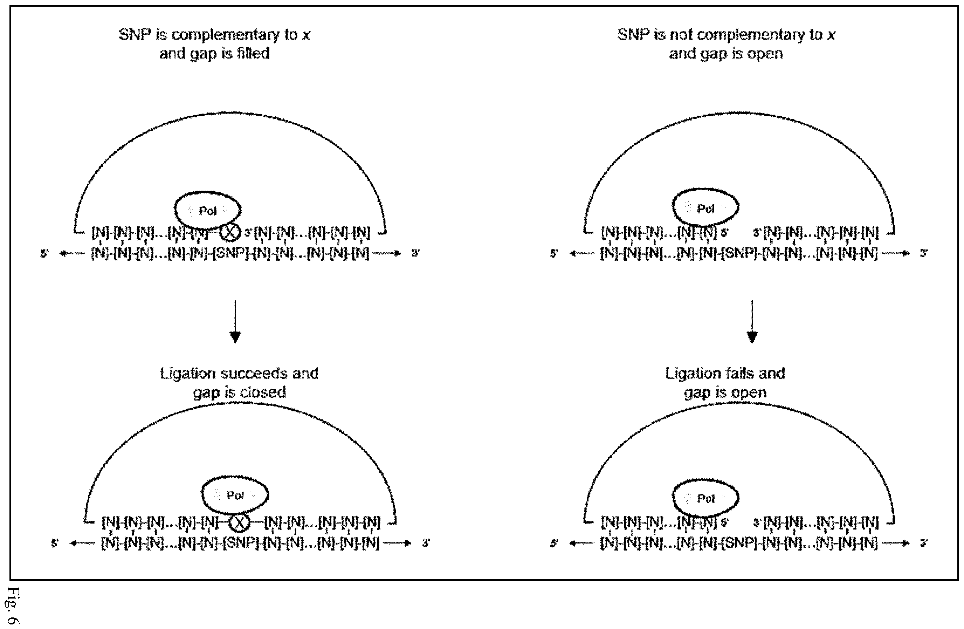

Figure 6 shows an example of the possible outcomes of ligation (in this example, by a ligation enzyme) for the two possible outcomes of Step 3

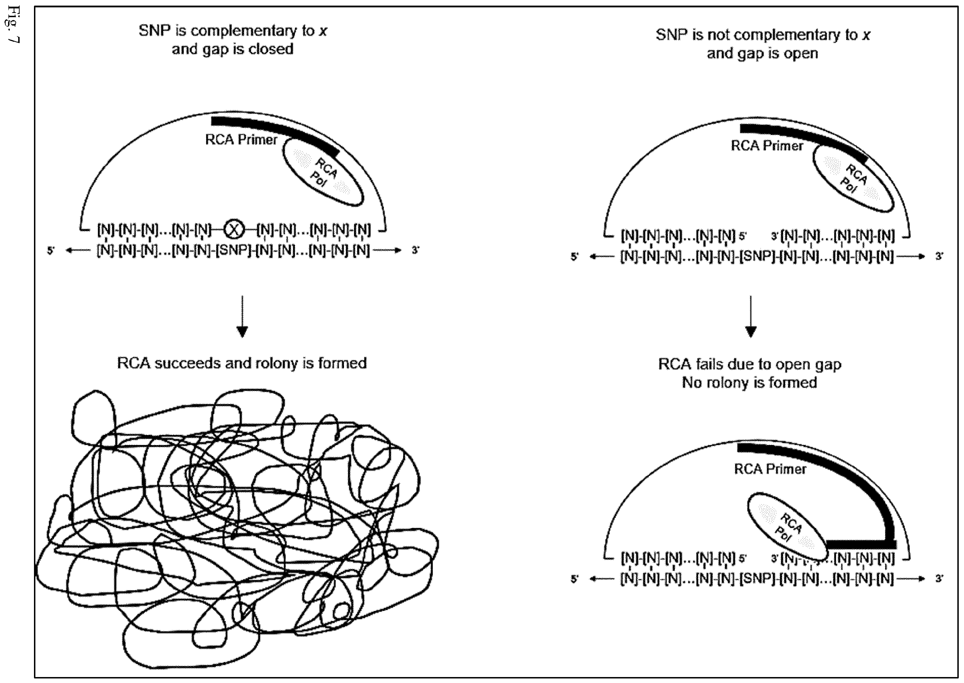

Figure 7 shows an example of the possible outcomes from RCA (in this example, performed by hybridizing an RCA primer and activating an RCA capable polymerase) for the possible outcomes from previous steps

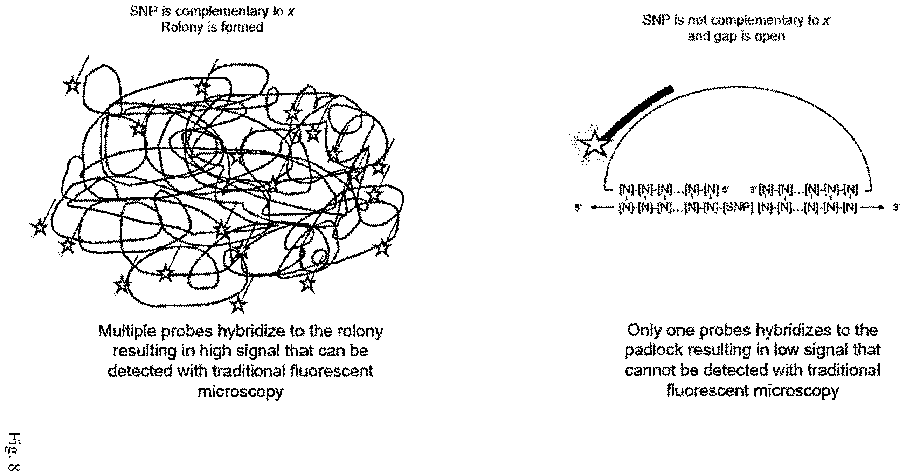

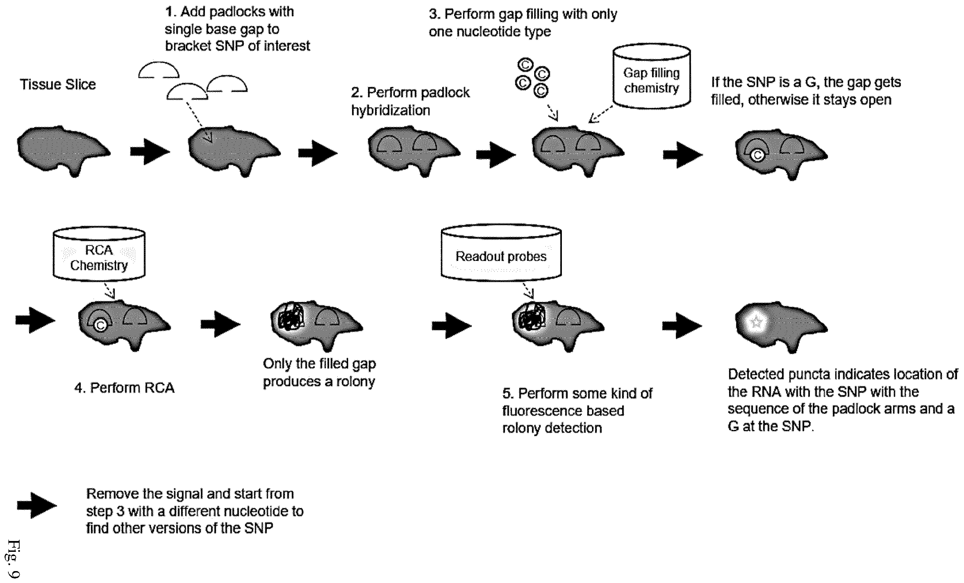

Figure 8 shows an example of the possible outcomes from rolony detection (in this example, performed by hybridizing a fluorescent probe capable of binding to a barcode sequence on the padlock) for the possible outcomes from previous steps.

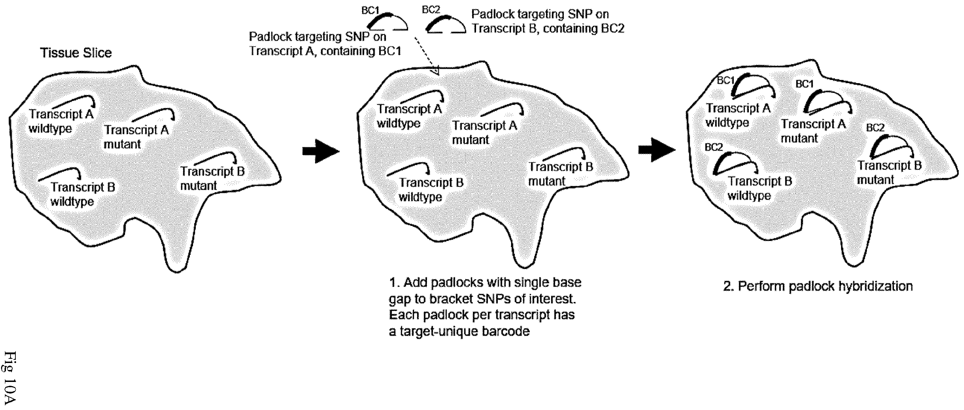

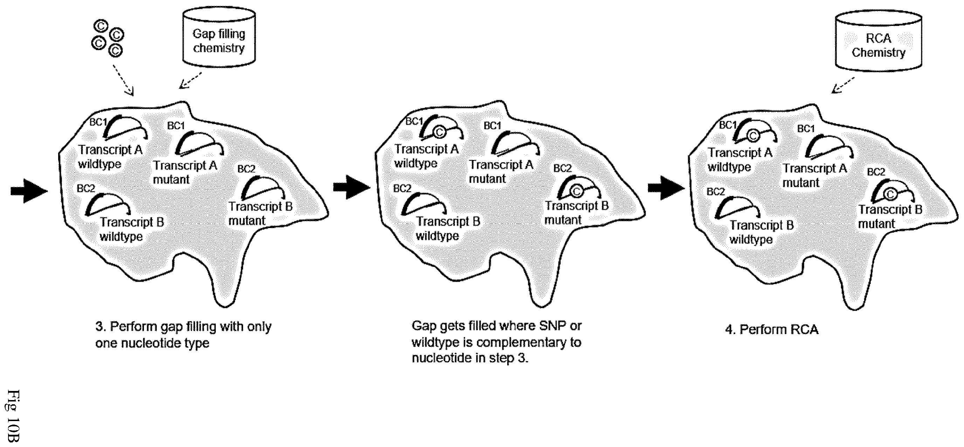

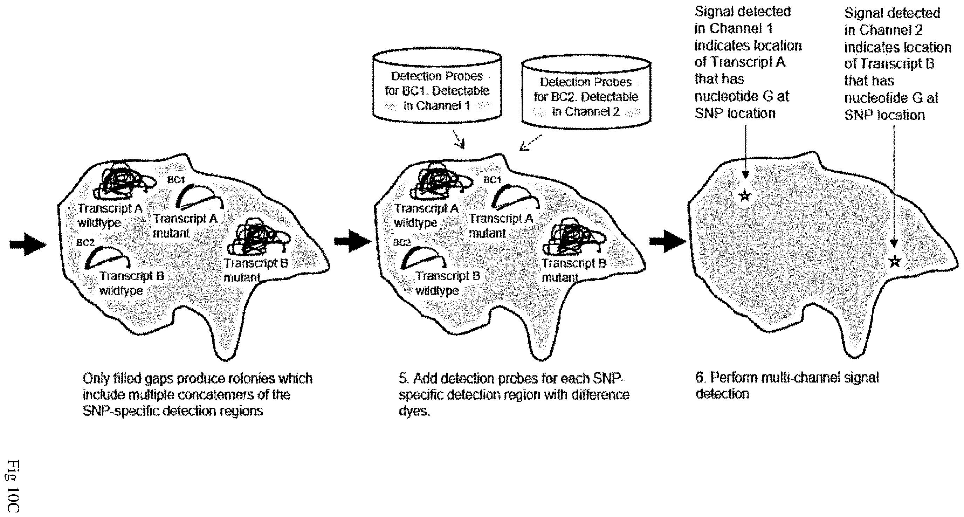

Fig. 10 shows and embodiment of the invention with several cycles of gap-filling

### DETAILED DESCRIPTION:

The workflow of the method of the invention prerequisites having knowledge of at least one SNP of interest that may be present in a RNA on a tissue sample.

This knowledge includes knowing the nucleotide sequences of 10 - 30, preferable at least 25 (to 30) nucleic acids in both the 5' and 3' directions of the SNPs of interest (sequences designated Um and Dn respectively)

The method of the invention involves performing a fluorescent based rolony detection method by hybridization of a fluorescently labelled probe with an oligonucleotide complementary to the detection region. The fluorescent detection method is sensitive enough to detect amplified signals from a rolony, but not sensitive enough to detect signal coming from single padlocks.

Multiplexing using padlocks with target specific barcode binding regions is known and described for example in EP3916388A1 " METHOD COMBINING SINGLE CELL GENE EXPRESSION MAPPING AND TARGETED RNA OR c-DNA SEQUENCING USING PADLOCK OLIGONUCLEOTIDES COMPRISING A BARCODE REGION"; EP22178553.8, "METHOD OF SPATIAL SEQUENCING OF GENES FROM TISSUE USING PADLOCKS WITH GAPS ON SUBSTRATE" and EP4039822A1 "METHOD COMBINING IN SITU TARGET CAPTURE AND SPATIAL UNIQUE MOLECULAR IDENTIFIER (SUMI) IDENTIFICATION WITH IN VITRO SEQUENCING FOR HIGH DENSITY SPATIAL MULTIOMICS"

The method of the invention involves one or more of the following steps
1. Hybridizing padlocks, with a single base gap at the targeted SNP location in the transcriptome, on a tissue sample
2. Performing an extension and ligation to fill the padlock gap while only making a single nucleotide type available for the extension reaction Adenine (A), Cytosine (C), Thymine (T), Guanine (G) or Uracil (U). If the available nucleotide type is complementary to a SNP mutation present, the padlock gap fill would be successful, otherwise the padlock gap will remain open.
3. Attempt a rolling circular amplification (RCA) reaction of the sample. In cases where the gap fill in step 2 was successful, amplified RCA products will form. In cases where the gap fill in step 2 was not successful, RCA products will not form.
4. Perform a fluorescent labelling to identify RCA products (i.e. the rolonies). Labelling can either be done through a pre-determined sequence on the padlock (i.e. a known barcode sequence) or through any other method to label ssDNA.
5. Determine the spatial locations and counts of the amplified RCA products, via fluorescent microscopy, to identify where the SNP's with mutations complementary to the nucleotide introduced in step 2 are present on the sample.
6. Optional repeat Steps 2 through 6 with a different nucleotide introduced in step 2 to identify other possible variants

The method of the invention may optionally involve further one or more of the following steps
- Identifying the locations of possible rolonies through image analysis. The detected rolonies will indicate location where the SNP of interest in present and has the mutation variant complementary to the single nucleotide type in step 3.
- Optionally, performing a reaction to remove fluorescent labelling introduced in step 6.
- Optional repeating the steps different nucleotides to identify other possible variants of an (unknown) SNP
- Optionally, removing the padlocks created on the sample and sequentially repeating the steps for multiple iterations, where the single nucleotide x in step 3 is not the same nucleotide used in a previous iteration.
- Removing the detection probe is removed from the rolony
- Removing the dye chemically or enzymatically from the bound detection probe.
- Replication of the single strand circular template non-selectively using oligonucleotides complementary to the padlock primer region as priming site for the rolling circle amplification polymerase.
- Providing a plurality of detection probes capable of binding to at least one detection region of the oligonucleotides
- Providing a plurality of detection probes capable of binding to different portion of the detection region is provided in a sequential manner wherein a first detection probe or the dye is removed after detection before the next detection probe

In a embodiment of the method, the SNP per se is known, and while the method of the invention involves technically sequencing, it is more directed detect the presence or absence of the SNP.

In this case, only one (i.e. the nucleotide of the known mutation) of the nucleotides A,T, C, G or U is provided for filling the gap of the padlock with the nucleotide. If the known or suspected SNP is present, a single strand circular template is generated. If the known SNP is not present, the padlock stays with an open gap and no single strand circular template is generated.

By providing a polymerase capable of rolling circle amplification / using the primer region of the oligomer, the single strand circular templates (if present) is multiplied into a plurality of concatemers forming a rolony. Detection of the rolonies is then performed by binding to the detection regions at least one detection probe comprising a dye and a sequence of nucleotides complementary to the detection regions. Obviously, the detection probes bind to the non-multiplied open padlocks too, but the signal generated from these has a too low intensity to be detected and/or can easily designated as irrelevant.

With this embodiment, presence or absence of the SNP and in the end presence or absence of a disease can be detected.

In a second embodiment of the method, the SNP is not known and the method of the invention is more directed to sequencing of the SNP, but obviously gives information about presence or absence of an SNP.

In this case, at least one (at most all) of the nucleotides A, T, C, G, or U is provided in a sequential manner for filling the gap of the padlock with the nucleotide. In each of these sequences, if the provided nucleotide fills the gap, a single strand circular template can be generated. Of course, if the provided nucleotide does not fill the gap, the padlock stays with an open gap and no single strand circular template is generated.

In each of these sequences, by providing a polymerase capable of rolling circle amplification / using the primer region of the oligomer, the single strand circular templates (if present) is multiplied into a plurality of concatemers forming a rolony. In each of these sequences, detection of the rolonies is then performed by binding to the detection regions at least one detection probe comprising a dye and a sequence of nucleotides complementary to the detection regions. Obviously, the detection probes bind to the non-multiplied open padlocks too, but the signal generated from these has a too low intensity to be detected and/or can easily designated as irrelevant.

In this embodiment, presence or absence of the SNP, the nucleotide sequence of the SNP and in the end presence or absence of a disease can be detected.

In a third embodiment of the method, multiple SNPs (either known or suspected or unknown or any combination thereof) are interrogated in a multiplexed parallel manner.

In this case, multiple padlocks targeting different SNPs are hybridized. Each padlock contains at least one detection region unique to the target SNP. At least one (at most all) of the nucleotides A, T, C, G, T or U is provided in a sequential manner for filling the gaps of the padlocks with the nucleotide. In each of these sequences, if the provided nucleotide fills any gaps, a single strand circular template can be generated. Of course, if the provided nucleotide does not fill a gap, the padlock stays with an open gap and no single strand circular template is generated.

In each of these sequences, by providing a polymerase capable of rolling circle amplification / using the primer region of the oligomer, the single strand circular templates (if present) are multiplied into a plurality of concatemers forming a rolony. In each of these sequences, detection of the rolonies is then performed by binding to the detection regions at least one detection probe comprising a dye and a sequence of nucleotides complementary to the detection regions. Obviously, the detection probes bind to the non-multiplied open padlocks too, but the signal generated from these has a too low intensity to be detected and/or can easily designated as irrelevant.

In this embodiment, detection probes having different dyes (or dyes having sufficiently different absorption and/or emission spectra) need to be used. The different dyes are aligned with the different SNP-specific detection regions.

With this embodiment, presence or absence of multiple SNPs, their sequences and in the end presence or absence of a disease can be detected.

### USE OF THE METHOD

The method can be used to detect the presence or absence known or suspected SNPs in a sample. In a medical application, the method according to the invention can be used for detection of cancer in the tissue sample.

For example, T regulatory cells (Tregs) and related-cytokines are effectively engaged in the process of tumor immune escape and functionally inhibit immune response against the tumor. Foxp3 gene single nucleotide polymorphism (SNP) (rs3761548) might be involved in the progression of gastric adenocarcinoma through influencing Tregs function and the secretion of immunomodulatory cytokines IL-35, IL-10, and TGF-β levels (Cytokine Volume 138, February 2021, 155351)

Another example is a rare SNP( rs188140481[A] ) that confers greater risk of prostate cancer. A rare 8q24 single nucleotide polymorphism (SNP) predisposes North American men to prostate cancer and possibly more aggressive disease (Boris Grin,Stacy Loeb,Kim Roehl,Phillip R. Cooper,William J. Catalona,Brian T. Helfand, First published: 20 June 2014,
https://doi.org/10.1111/bju.12847)

BRCA1 and BRCA2 genes mutations associated with risk of breast and ovarian cancer . SNP rs6138178 in SNRP gene rs6602595 in CAMK1D gene displayed the strongest associations in BRCA1 carriers whereas rs12652447 in FBXL7 gene showed the strongest associations in BRCA2 carriers and cumulative risk of cancer for example (Hum Mol Genet. 2010 Jul 15; 19(14): 2886-2897)

## Claims

1. A method for determining the sequence of single nucleotide polymorphisms (SNP) of at least one RNA strand on tissue samples **characterized by** the steps
a. providing an oligonucleotide comprising 50 - 1000 nucleotides, having a 3' end and a 5' end each with 10 to 30 nucleic acids complementary to the nucleic acids of the RNA from the SNP towards the 3' end and the 5' end of the RNA respectively, a primer region and at least one detection region, each having 2 to 20 nucleotides
b. hybridizing the oligonucleotide with its 5' and 3' ends to complementary parts of the at least one RNA strand to create a padlock with a gap of one nucleotide between the 5' and the 3' end of the oligonucleotide thereby bracketing the SNP
c. performing for at least one of the nucleotides Adenine (A), Thymine (T), Cytosine (C), Guanine (G) and Uracil (U) a sequence comprising (i) filling the gap of the padlock with the at least one nucleotide, thereby generating a single strand circular template; (ii) multiplying the single strand circular template by a polymerase capable of rolling circle amplification using the primer region into a plurality of concatemers forming a rolony; (iii) binding to the detection regions at least one detection probe comprising a dye and a sequence of nucleotides complementary to the detection regions
d. detecting the dye of the detection probes, thereby determining the sequence of the single strand circular template

2. Method according to claim 1 or 2 **characterized in that** by detecting the detection probes, the spatial location of the rolonies is determined.

3. Method according to claim 1 or 2 **characterized in that** the detection probe is removed from the rolony.

4. Method according to claim 1 or 2 **characterized in that** the dye is chemically or enzymatically removed from the bound detection probe.

5. Method according to any of the claims 1 to 5 **characterized in that** the single strand circular template is replicated non-selectively using oligonucleotides complementary to the padlock primer region as priming site for the rolling circle amplification polymerase.

6. Method according to any of the claims 1 to 8 **characterized in that** a plurality of detection probes capable of binding to at least one detection region of the oligonucleotides is provided.

7. Method according to any of the claims 1 to 9 **characterized in that** a plurality of detection probes capable of binding to different portion of the detection region is provided in a sequential manner wherein a first detection probe or the dye is removed after detection before the next detection probe is provided.

8. Use of the method according to any of the claims 1 to 7 for determination if one or more known single nucleotide polymorphisms (SNP) of RNA strands on a tissue sample are present.

9. Use of the method according to claim 8 for detection of cancer in the tissue sample.
